(19) Europäisches Patentamt European Patent Office Office européen des brevets

(11) **EP 4 070 077 B1**

(12) **EUROPÄISCHE PATENTSCHRIFT**

(45) Veröffentlichungstag und Bekanntmachung des Hinweises auf die Patenterteilung:
**25.12.2024   Patentblatt 2024/52**

(21) Anmeldenummer: **20821150.8**

(22) Anmeldetag: **04.12.2020**

(51) Internationale Patentklassifikation (IPC):
**G01N 21/64** *(2006.01)*     **A61B 5/145** *(2006.01)*
**A61B 5/1486** *(2006.01)*     **G01N 33/66** *(2006.01)*
**A61B 5/00** *(2006.01)*     *G01N 21/77* *(2006.01)*

(52) Gemeinsame Patentklassifikation (CPC):
**G01N 21/645; A61B 5/0071; A61B 5/14532;**
**A61B 5/14865; A61B 5/6852; G01N 33/66;**
G01N 2021/6434; G01N 2021/6471;
G01N 2021/6484; G01N 2021/772;
G01N 2021/7786

(86) Internationale Anmeldenummer:
**PCT/EP2020/084747**

(87) Internationale Veröffentlichungsnummer:
**WO 2021/110977 (10.06.2021 Gazette 2021/23)**

(54) **ANORDNUNG ZUM BETRIEB EINES BIOSENSORS SOWIE ANORDNUNG ZUR BESTIMMUNG DES GLUKOSEGEHALTS IM BLUT**

ARRANGEMENT FOR THE OPERATION OF A BIOSENSOR AND SYSTEM FOR THE DETERMINATION OF A GLUCOSE CONTENT IN BLOOD

DISPOSITIF POUR LE FONCTIONNEMENT D'UN BIOCAPTEUR ET DISPOSITIF POUR LA DÉTERMINATION DE LA TENEUR EN GLUCOSE DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.12.2019   DE 102019133365**

(43) Veröffentlichungstag der Anmeldung:
**12.10.2022   Patentblatt 2022/41**

(73) Patentinhaber: **EyeSense GmbH**
**63762 Großostheim (DE)**

(72) Erfinder:
• **MÜLLER, Ralf**
**09648 Mittweida (DE)**
• **MÜLLER, Achim**
**63762 Großostheim (DE)**
• **KRIVANÉK, Roland**
**63743 Aschaffenburg (DE)**

(74) Vertreter: **Engel, Christoph Klaus**
**PATENTSCHUTZengel**
**Marktplatz 6**
**98527 Suhl (DE)**

(56) Entgegenhaltungen:
US-A- 4 496 211     US-A- 5 341 805
US-A- 5 999 673     US-A1- 2004 072 358
US-A1- 2008 188 725

**Beschreibung**

Die vorliegende Erfindung betrifft eine faseroptische Anordnung zum Betrieb eines Biosensors. Der Biosensor eignet sich insbesondere, wenngleich nicht ausschließlich zur Bestimmung des Glukosegehalts im Blut. Die Erfindung betrifft daher ebenso eine Anordnung zur Bestimmung des Glukosegehalts im Blut.

Optische Sensoren kommen unter anderem zur Auswertung von Fluoreszenzstrahlung zum Einsatz. In vielen Fällen wird dabei durch eine optische Anordnung sowohl eine Anregungsstrahlung bereitgestellt als auch die von einem geeigneten Leuchtstoff emittierte Strahlung ausgewertet. Die Intensität der emittierten Fluoreszenzstrahlung kann dabei ein Maß für eine zu überwachende Größe sein.

So beschreibt beispielsweise die DE 10 2015 101 847 B4 eine Anordnung zur Untersuchung einer mittels elektromagnetischer Strahlung anregbaren Probe. Zur Trennung der Anregungsstrahlung von der emittierten Messstrahlung besitzt die Anordnung einen ersten dichroitischen Strahlteiler mit einem ersten und einem zweiten Prisma, welche an ihren Basisflächen miteinander verbunden sind, und mit einer zwischen den Basisflächen der beiden Prismen angeordneten dichroitischen Schicht. Eine Lichtquelle liefert eine zur Anregung der Probe geeignete elektromagnetische Strahlung, welche in die Eintrittsfläche des ersten Prismas eingekoppelt wird. Ein Teil der Strahlung wird an der dichroitischen Schicht reflektiert in Richtung zur Probe, die hinter einer Austrittsfläche des ersten Prismas positioniert ist. Ein Detektor dient der Erfassung der von der Probe emittierten, durch den Strahlteiler durchgeleiteten und diesen an einer Messfläche verlassenden elektromagnetischen Messstrahlung. Ein Nachteil solcher Anordnungen besteht in dem empfindlichen und vergleichsweise großen Aufbau, der beispielsweise eine Verwendung in Alltagssituationen und durch ungeschultes Personal nicht möglich erscheinen lässt.

In Müller AJ, Knuth M, Nikolaus KS, Krivänek R, Küster F, Hasslacher C. "First clinical evaluation of a new percutaneous optical fiber glucose sensor for continuous glucose monitoring in diabetes". Journal of Diabetes Science and Technology 2013; 7(1):13-23. 2013 Jan 01, wird eine Anordnung mit einem Glukose empfindlichen Sensor beschrieben, der als Biosensor am Ende einer optischen Faser ausgebildet ist. Dafür sind an der Faser ein oder mehrere fluoreszierende Leuchtstoffe angeordnet, die von einer Anregungsstrahlung angeregt werden. Die Anregungsstrahlung wird von einer LED geliefert und über eine Linse in die Faser eingekoppelt. Die Intensität der emittierten Fluoreszenzstrahlung ist in dem konkreten Anwendungsfall abhängig von dem Glukosegehalt im Blut in einem Gewebe, in welches die Faser implantiert werden kann. Die emittierte Fluoreszenzstrahlung wird in der Faser zu einem Detektor geleitet und von diesem ausgewertet. Grundsätzlich eignet sich ein solcher Biosensor für eine quasikontinuierliche Messung des Blutzuckergehalts, sodass die gelieferten Werte beispielsweise einer Insulin-Dosiereinheit zugeführt werden können, um einem Patienten bedarfsgerecht Insulin zuzuführen. Allerdings sind die Zuleitung der Anregungsstrahlung und die Auswertung der vom Biosensor gelieferten Fluoreszenzstrahlung technisch schwierig in kleinsten Einheiten umsetzbar, sodass der Patient gezwungen wäre, eine größere Anordnung mitzuführen.

Die DE 694 08 976 T2 beschreibt einen Glukosemonitor, der eine Lichtquelle, einen Sensor und einen Prozessor enthält. Die Lichtquelle sendet Anregungslicht aus, das auf die Probe gerichtet ist, um die Fluoreszenz von Glucose in der Probe zu induzieren. Das Anregungslicht bewirkt, dass die Probe Rücklicht erzeugt, das fluoreszierendes Licht enthält, das von einer beliebigen Glukose in der Probe erzeugt wird. Der Sensor überwacht das zurückkehrende Licht und erzeugt zwei Signale, die die Intensität des Lichts innerhalb zweier spektraler Wellenlängenbänder darstellen. Das erste Signal gibt die Intensität des zurückkehrenden Lichts mit einer Wellenlänge innerhalb eines ersten Wellenlängenbandes an. Das zweite Signal gibt die Intensität des Lichts innerhalb eines zweiten Wellenlängenbandes an. Der Prozessor verarbeitet die beiden elektrischen Signale, um die Glukosekonzentration in der Probe zu bestimmen. Als optische Komponenten kommen Fasern oder Wellenleiter zur Lichtleitung, ein dichroitisches Filter zum Trennen des Anregungslichts vom Rücklicht, eine Blende mit einem Schlitz und ein Prisma zum Einsatz.

Die US 4,344,438 offenbart eine Lösung zur invivo-Messung der Konzentration von Plasmabestandteilen mit niedrigem Molekulargewicht. Gezeigt wird ein fluoreszenzbasierter Glukosesensor mit einer Kathetermesskammer, wobei die Erfassungseinrichtung und die Lichtquelleneinrichtung über optische Fasern in optischem Kontakt mit der Kammer stehen. Dabei gelangt das Anregungslicht von der Quelle durch ein Filter zu einem halbversilberten Spiegel und wird dann auf das Ende einer optischen Faser fokussiert. In einer Ausführungsform können diese Komponenten miniaturisiert werden, indem eine LED für die Lichtquelle und eine Fotodiode für den Lichtdetektor verwendet werden und die gesamte Anordnung in den Körper implantiert wird.

Die WO 2014/116597 A1 zeigt ein optisches System zur Erfassung von Fluoreszenzlicht einer biologischen Probe. Ein benutzter Optischer Koppler umfasst eine Faser zur Kontaktierung eines fluoreszierenden Körpers, eine Leuchtdiode, mehrere Filter und eine Fotodiode.

Die EP 2 989 975 A1 zeigt einen faseroptischen Glukosesensor, umfassend ein Element mit proximalen und distalen Endbereichen, wobei der proximale Endbereich zum Koppeln mit einer optischen Vorrichtung konfiguriert ist, die eine Anregungslichtquelle und einen Detektor umfasst. Das distale Ende kann in einem Blutgefäß positioniert werden und umfasst einen Hohlraum und eine reflektierende Oberfläche, wobei der Hohlraum ein Indikatorsystem umfasst.

Die DE 43 22 734 A1 und die DE 43 41 086 A1 zeigen einen optischen Y-Koppler, bestehend aus einem durchgehenden Lichtwellenleiter mit Polymermantel und einem seitlich einmündenden Lichtwellenleiter mit Polymermantel. Der durchgehende Lichtwellenleiter verläuft an der Einmündungsstelle gerade, der seitlich einmündende Lichtwellenleiter unter einem Winkel α, der kleiner ist als der Grenzwinkel für die Totalreflexion in dem durchgehenden Lichtwellenleiter. Der durchgehende Lichtwellenleiter weist einen Durchmesser von 50 bis 6000 μm auf.

Die US 6,553,164 zeigt einen wellenleiterbasierten Y-Koppler, der aus 4 einzelnen Teilelementen besteht.

Die DE 694 14 139 T2 beschreibt eine Kopplungsanordnung zwischen einer multimodalen Lichtquelle und einer optischen Faser mittels einer Zwischenfaser, welche als optische Mehrmoden-Zwischenfaser ausgebildet ist. Die Zwischenfaser weist einen Teilbereich mit dem Querschnitt und einen verjüngenden Teilbereich auf. Mittels der sich verjüngenden Zwischenfaser wird ein Y-Koppler ausgebildet.

Die US 2004/072358 A1 beschreibt eine Glukoseerfassungseinrichtung, welche einen Sensor, eine Lichtquelle und einen Fotodetektor besitzt, die durch eine optische Faser miteinander verbunden sind. Weiterhin wird ein Y-Koppler genutzt, der durch eine sich in zwei Stränge aufteilende optische Faser gebildet wird.

Ausgehend von der US 2004-072358 A1 besteht eine Aufgabe der vorliegenden Erfindung darin, eine verbesserte Anordnung zum Betrieb eines Biosensors bereitzustellen. Die Anordnung soll sowohl die Anregungsstrahlung liefern als auch in der Lage sein, die vom Sensor erzeugten Messsignale mit hoher Empfindlichkeit zu detektieren. Dabei sollen ein kleiner, integrierbarer Aufbau eine sichere mechanische Anbindung an den Biosensor und eine hohe optische Zuverlässigkeit erreicht werden. Darüber hinaus wird eine Aufgabe darin gesehen, eine verbesserte Anordnung zur Bestimmung des Glukosegehalts, insbesondere im Blut bereitzustellen, welche eine mobile, quasikontinuierliche Messung des Glukosegehalts gestattet. Diese und weitere Aufgaben werden von einer Anordnung zum Betrieb eines Biosensors gemäß dem beigefügten Anspruch 1 gelöst.

Die erfindungsgemäße Anordnung zum Betrieb eines Biosensors besitzt eine Anregungslichtquelle, welche mindestens eine Anregungsstrahlung für den Biosensor erzeugt. Die Anordnung umfasst weiterhin eine Koppelfaser, an deren Eintrittsfläche die Anregungsstrahlung eingekoppelt wird. Darüber hinaus ist ein optischer Y-Koppler vorgesehen, der einen Anregungsarm, welcher an die Austrittsfläche der Koppelfaser angeschlossen ist, einen Detektorarm, welcher an einen optischen Detektor angeschlossen ist, und einen Sensorfuß, welcher an den Biosensor anschließbar ist, besitzt. Der Detektorarm und der Sensorfuß weisen vorzugsweise eine gemeinsame Hauptstrahlachse auf. Die Strahlachse des Anregungsarms verläuft zur Hauptstrahlachse des Detektorarms in einem Winkel im Bereich 5° bis 70°, vorzugsweise 5° bis 30°. Der Anregungsarm besitzt eine langgestreckte, konische Form, wobei der Durchmesser des Anregungsarms an der Eintrittsstelle in den Detektorarm weniger als zwei Drittel des Durchmessers des Detektorarms an diesem Verbindungspunkt beträgt, vorzugsweise weniger als die Hälfte, besonders bevorzugt weniger als ein Drittel des Durchmessers des Detektorarms.

Vorzugsweise ist die Anregungslichtquelle durch einen LED-Chip gebildet, welcher eine Anregungsstrahlung von beispielsweise 595 nm emittiert. Die Anregungsstrahlung ist in Wellenlänge und optischer Leistung an den Biosensor angepasst und kann beispielsweise auch zwei Wellenlängen enthalten, wenn dies für den jeweiligen Anregungszweck gewünscht ist. Bevorzugt ist die Emissionsebene des LED-Chips von der Eintrittsfläche der Koppelfaser in einem Abstand positioniert, der das 0,1 bis 10-fache des Durchmessers der Koppelfaser beträgt.

Gemäß einer bevorzugten Ausführungsform ist die Anregungslichtquelle ein planer, diffus abstrahlender Emitter. Vorzugsweise erfolgt die Emission aus einer dünnen Schicht heraus, wie es beispielsweise bei einer Dünnfilm-LED geschieht. Besonders bevorzugt ist die Anregungslichtquelle daher durch eine Dünnfilm-LED gebildet.

Die Koppelfaser ist bevorzugt eine PMMA-Faser oder eine Saphir-Faser, die beispielsweise eine sphärische, asphärische oder plane Eintrittsfläche besitzt, welche der Anregungslichtquelle gegenüberliegt. Die Koppelfaser besitzt vorzugsweise einen über ihre Länge konstanten Durchmesser im Bereich 0,1 bis 2 mm, weiter bevorzugt 0,3 bis 0,7 mm, besonders bevorzugt 0,5 mm, und eine Länge, die bevorzugt das 7-fache bis 13-fache des Abstands zwischen der Anregungslichtquelle und der Eintrittsfläche der Koppelfaser beträgt, beispielsweise 5 bis 15 mm, besonders bevorzugt 10 mm. Die Eintrittsfläche der Koppelfaser ist von der Anregungslichtquelle vorzugsweise etwa 0,8 bis 1,2 mm, besonders bevorzugt etwa 1 mm beabstandet, wobei der Anregungsstrahl in diesem Abstand vorzugsweise in Luft verläuft. Dieser Abstand hat zur Folge, dass in die Koppelfaser nur "flache Strahlen" eingekoppelt werden, die also nur einen kleinen Winkel gegenüber der Zentralachse der Koppelfaser aufweisen. Die emittierende Fläche der Anregungslichtquelle entspricht vorzugsweise 10 bis 60 % der Querschnittsfläche der Koppelfaser.

Eine vorteilhafte Ausführungsform zeichnet sich dadurch aus, dass die Koppelfaser in axialer Längsrichtung keine Krümmung aufweist, d. h. ihre Längsachse geradlinig verläuft. Beispielsweise kann die Koppelfaser dazu aus einem starren Material bestehen oder in einer nicht-gekrümmten Hülse geführt sein.

Gemäß einer bevorzugten Ausführungsform ist die Austrittsfläche der Koppelfaser unter Zwischenschaltung eines Cut-off-Filters an den Anregungsarm des optischen Y-Kopplers angekoppelt. Durch diesen Filter können aus der Anregungsstrahlung die Wellenlängen herausgefiltert werden, welche den vom Biosensor emittierten Fluoreszenzwellenlängen entsprechen, sodass diese am Detektor leichter als Messsignale detektiert werden können. Der Cut-off-Filter ist beispielsweise als ein Trägerglas mit aufgebrachten Filterschichten gebildet. Die Austrittsfläche der Koppelfaser kann an diesem Trägerglas angeklebt sein, wobei die Klebung auch direkt auf die Filterschichten möglich ist.

Eine besonders bevorzugte Ausführungsform zeichnet sich durch eine speziell angepasste Strahlführung aus, die eine optimierte Nutzung der vorhandenen optischen Leistung gestattet. Dazu werden in die genannte Koppelfaser die bereits erwähnten "flachen Strahlen" eingekoppelt. Unter "flachen Strahlen" werden Strahlen mit einer kleinen Winkelabweichung in Ausbreitungsrichtung verstanden. Die Verwendung solcher Strahlen gestattet vor allem auch den effizienten Einsatz des Cut-off-Filters, der bereits aus der Anregungsstrahlung bestimmte Wellenlängen ausschneidet, die dann das Messergebnis nicht mehr verfälschen. Anzustreben ist das Auftreffen der Anregungsstrahlung möglichst senkrecht auch die Oberfläche des Cut-off-Filters, was erreicht wird, wenn sich die Anregungsstrahlung in möglichst geringer Winkelabweichung von der Längsachse der Koppelfaser in dieser ausbreitet und daher mit möglichst kleiner Winkelabweichung zum Flächenlot des Cut-off-Filters auf diesen auftrifft. Bevorzugt verläuft die Anregungsstrahlung in der Koppelfaser mit einem Winkel <40° zum Flächenlot des Cut-off-Filters bzw. zur Seelenachse der Koppelfaser, besonders bevorzugt mit einem Winkel <30°, insbesondere <25°.

Eine vorteilhafte Ausführungsform der Anordnung zeichnet sich dadurch aus, dass der Durchmesser des Anregungsarms von seiner Eintrittsfläche, welche optisch an die Austrittsfläche der Koppelfaser angekoppelt ist, in Richtung zu der Verbindungsstelle mit dem Detektorarm kontinuierlich abnimmt (sogenannte Tapered Fiber). Der Anregungsarm besitzt damit die Form eines lang gestreckten Kegelstumpfes. An der Verbindungsstelle, also an der Position, an welcher Anregungsarm, Detektorarm und Sensorfuß des Y-Kopplers zusammenlaufen, ist der Durchmesser des Koppelarms somit deutlich kleiner als an seiner Eintrittsfläche. Vorzugsweise reduziert sich der Durchmesser des Anregungsarms entlang seiner Längserstreckung um mehr als die Hälfte. Insbesondere besitzt der Anregungsarm an der Verbindungsstelle mit dem Detektorarm einen Durchmesser im Bereich 0,1 bis 0,2 mm. Die Verjüngung des Anregungsarms dient vor allem der Ausbildung eines optischen Ventils, welches die Einkopplung der Anregungsstrahlung in den Sensorfuß ermöglicht, gleichzeitig aber das unerwünschte Austreten der vom Biosensor zurückgegebenen Fluoreszenzstrahlung in den Anregungsarm minimiert.

Gemäß einer bevorzugten Ausführungsform befindet sich zwischen dem beschichteten Trägerglas, welches an die Austrittsfläche der Koppelfaser anschließt, und der Eintrittsfläche des Anregungsarms ein Farbglasstück, welches die Filterwirkung des Cut-off-Filters unterstützt. Das Farbglasstück ist in der Art eines Wellenleiters ausgebildet und kann ebenfalls eine konische Form aufweisen. Bei einem Farbglasstück handelt es sich um farbiges, optisches Filterglas, wie es z. B. von der Schott AG angeboten wird.

Vorzugsweise wird der Y-Koppler aus Kunststoff, insbesondere PMMA oder PC hergestellt. Dafür eignen sich beispielsweise 3D-Druckverfahren oder Spritzgussverfahren. Der Durchmesser des Detektorarms und des Sensorfußes sind vorzugsweise gleichbleibend, beispielsweise im Bereich 0,4 bis 0,6 mm, insbesondere 0,5 mm. Der Sensorfuß kann vorteilhaft in Längsrichtung eine Krümmung mit einem Winkel von etwa 90° aufweisen, um an seiner Austrittsfläche einfach an den Biosensor angeschlossen werden zu können, wenn dieser im Wesentlichen senkrecht zur Hautoberfläche eines Patienten in das Gewebe implantiert wurde.

Eine zweckmäßige Ausführungsform besitzt ein weiteres Farbglasstück in Wellenleiterform, welches zwischen dem Detektor und der Austrittsfläche des Detektorarms angeordnet ist. Dieses Farbglasstück erfüllt wiederum Filter unterstützende Funktionen. Weiterhin ist es vorteilhaft, wenn vor dem Detektor eine Linse und ein Filter angeordnet sind, um die vom Detektorarm kommende Fluoreszenzstrahlung zu kollimieren und eventuell noch enthaltene Anregungsstrahlung auszufiltern.

Der Detektor kann vorzugsweise als Fotodiode oder als ein ähnliches Bauteil gebildet sein. Sofern mehrere Fluoreszenzwellenlängen detektiert werden sollen, kann es vorteilhaft sein, einen Strahlteiler mit mehreren Fotodetektoren unterschiedlicher Empfindlichkeit zu kombinieren.

Erfindungsgemäß dient der Sensorfuß nicht nur der mechanischen Ankopplung an die Sensorfaser sondern auch der Strahlumlenkung. Vorzugsweise ist dafür im Sensorfuß eine torische Fläche ausgebildet.

Eine erfindungsgemäße Anordnung zur Bestimmung des Glukosegehalts, insbesondere im Blut, umfasst einen Biosensor sowie eine Anordnung zu dessen Betrieb gemäß einer der hier beschriebenen Ausführungsformen.

Weitere Vorteile und Einzelheiten der Erfindung ergeben sich aus der nachfolgenden Beschreibung bevorzugter Ausführungsformen, unter Bezugnahme auf die Zeichnung. Es zeigen:

Fig. 1 eine Prinzipskizze einer ersten Ausführungsform einer erfindungsgemäßen Anordnung zum Betrieb eines Biosensors;

Fig. 2 eine Prinzipskizze einer zweiten Ausführungsform der Anordnung zum Betrieb des Biosensors;

Fig. 3 einen optischen Strahlverlauf in einem Sensorfuß zur Strahlumlenkung und Einkopplung in die bzw. Auskopplung aus der Sensorfaser mit geringen optischen Verlusten.

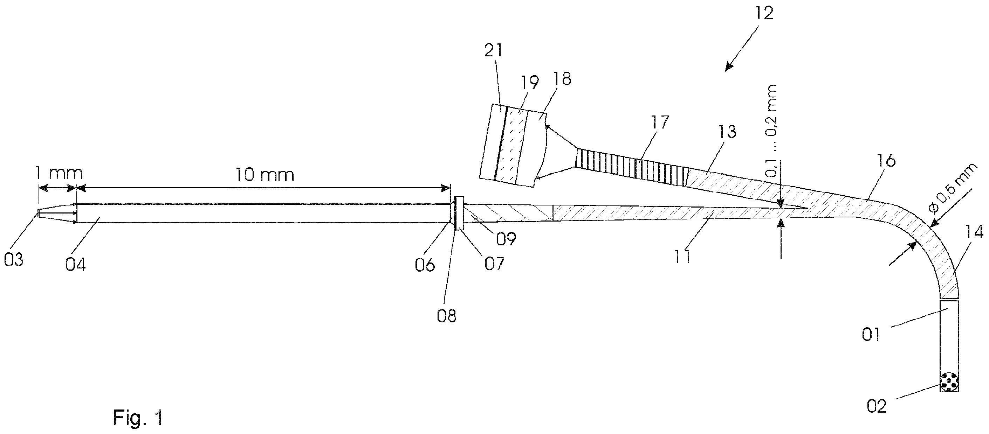

Fig. 1 zeigt in einer vereinfachten seitlichen Darstellung eine Anordnung zum Betrieb eines Biosensors. Der Biosensor ist hier als eine Sensorfaser 01 gebildet, die an ihrem freien Ende mit immobilisierten, Glukose-sensitiven Fluoreszenzleuchtstoffen 02 ausgerüstet ist. Bei Anregung mit einer Anregungsstrahlung emittieren diese Fluoreszenzleuchtstoffe eine Fluoreszenzstrahlung, deren Intensität abhängig ist von der Glukosekonzentration in einem Medium, in welchem das Ende des Biosensors positioniert ist. Das Medium kann insbesondere Blut sein.

Die Anordnung zum Betrieb des Biosensors besitzt einen LED-Chip 03 als Anregungslichtquelle, vorzugsweise eine Dünnfilm-LED. Der LED-Chip 03 emittiert die Anregungsstrahlung und koppelt diese mit flachem Winkel in eine Koppelfaser 04 ein. Der Winkel zwischen der Anregungsstrahlung und der Seele der Koppelfaser ist vorzugsweise <30°. Dazu ist die LED 03 bei der dargestellten Ausführungsform etwa 1 mm beabstandet von der Eintrittsfläche der Koppelfaser 04 positioniert. Die Eintrittsfläche der Koppelfaser kann als Freiformfläche, sphärische, asphärische oder plane Fläche gestaltet sein. Die Koppelfaser 04 hat beispielsweise einen Durchmesser von 0,5 mm und eine Länge von 10 mm. Die Austrittsfläche der Koppelfaser 04 ist mit einem Kleber 06 an einem Trägerglas 07 befestigt. Das Trägerglas 07 ist mit einer oder mehreren Filterschichten 08 ausgerüstet, um als Cut-off-Filter zu wirken und aus der Anregungsstrahlung die Wellenlänge der von den Fluoreszenzleuchtstoffen 02 emittierten Fluoreszenzstrahlung auszufiltern. In der Ausbreitungsrichtung der Anregungsstrahlung folgt auf das Trägerglas 07 ein erstes Farbglasstück 09, welches die Wirkung des Cut-off-Filters unterstützt. Das erste Farbglasstück 09 ist in Wellenleiterform gestaltet und schließt auf der gegenüberliegenden Seite an einen Anregungsarm 11 eines optischen Y-Kopplers 12 an. Der Anregungsarm 11 und das erste Farbglasstück 09 besitzen einen sich in Strahlungsrichtung verjüngenden Querschnitt. Der Durchmesser des Anregungsarms 11 an seinem der Koppelfaser 04 abgewandten Ende beträgt daher beispielsweise nur noch 0,1 bis 0,2 mm.

Der Y-Koppler 12 besitzt außerdem einen Detektorarm 13 und einen Sensorfuß 14. An einer Verbindungsstelle 16 tritt der Anregungsarm 11 in das im Übrigen vom Detektorarm 13 zum Sensorfuß 14 durchgehende Material ein. An der Verbindungsstelle 16 schließen der Detektorarm 13 und der Anregungsarm 11 einen Winkel im Bereich 5° bis 70°, bevorzugt 5° bis 30°, besonders bevorzugt etwa 15° ein. Die Hauptstrahlachse des Detektorarms 13 und des Sensorfußes 14 verlaufen in der gezeigten Ausführungsform koaxial. In abgewandelten Ausführungen können die Hauptstrahlachsen des Detektorarms und des Sensorfußes aber auch winklig zueinander verlaufen, beispielsweise mit einer Winkeländerung von 10° bis 40°. Der Sensorfuß 14 ist an seinem von der Verbindungsstelle 16 abgewandten Ende flexibel oder gekrümmt gestaltet, um an die Sensorfaser 01 angeschlossen werden zu können. Der Detektorarm 13 und der Sensorfuß 14 besitzen einen Durchmesser von beispielsweise 0,5 mm.

Die Anregungsstrahlung wird von dem LED-Chip 03 über die Koppelfaser 04, den Anregungsarm 11 und den Sensorfuß 14 in die Sensorfaser 01 geleitet und regt dort die Fluoreszenzleuchtstoffe 02 an. Die von diesen emittierte Fluoreszenzstrahlung läuft durch die Sensorfaser 01 zurück zum Sensorfuß 14 des Y-Kopplers 12 und dann zum größten Teil in den Detektorarm 13. An dem von der Verbindungsstelle 16 abgewandten Ende des Detektorarms 13 schließt sich ein zweites Farbglasstück 17 an, welches in Wellenleiterform gebildet ist. Zur Kollimation der an der Austrittsfläche des zweiten Farbglasstücks 17 austretenden Fluoreszenzstrahlung ist eine optische Linse 18 vorgesehen, der ein weiterer Filter 19 folgt, um nur die Fluoreszenzstrahlung zu einem nachfolgenden Detektor 21 passieren zu lassen.

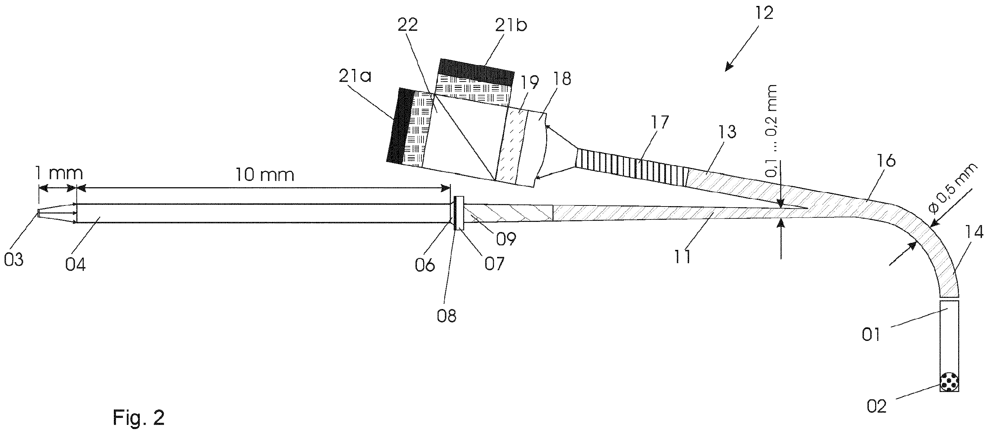

Fig. 2 zeigt eine abgewandelte Ausführungsform der Anordnung zum Betrieb des Biosensors, die zunächst mit dem Aufbau gemäß Fig. 1 weitgehend übereinstimmt. Ein Unterschied besteht darin, dass der Detektor in zwei Teildetektoren 21a und 21b geteilt ist, welche der Detektion unterschiedlicher Wellenlängen der Fluoreszenzstrahlung dienen. Dazu befindet sich in Strahlrichtung hinter dem Filter 19 ein Strahlteiler 22, welcher die Fluoreszenzstrahlung in Abhängigkeit von der Wellenlänge in zwei Teilstrahlen zerlegt, die dann den jeweiligen Teildetektoren 21a, 21b zugeleitet werden. Das zweite Farbglasstück 17 unterstützt die Wirkung des Filters 19.

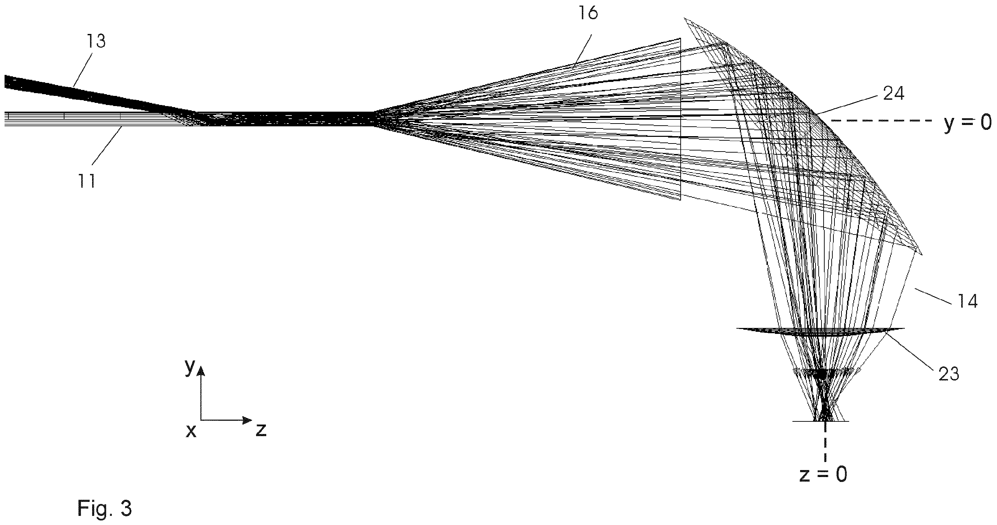

Fig. 3 zeigt beispielhaft den optischen Strahlverlauf im Anregungsarm 11, im Detektorarm 13, im Bereich der Verbindungsstelle 16 und vor allem im Sensorfuß 14. Der Sensorfuß 14 dient zur Strahlumlenkung und Einkopplung in die bzw. Auskopplung aus der Sensorfaser und schafft gleichzeitig der Möglichkeit eines Abstandes von mehreren 100 μm zwischen dem Sensorfuß und der Sensorfaser, wodurch sich die Positionierung erheblich vereinfacht. Wie bereits aus den Figuren 1 und 2 ersichtlich ist, wird der Sensorfuß 14 vorzugsweise gekrümmt bzw. gebogen ausgeführt, um einfach an die Sensorfaser 01 angeschlossen werden zu können und eine Strahlumlenkung zu realisieren. Die Eintrittsfläche am Anregungsarm 11 und die Austrittsfläche am Sensorfuß 14 stehen dadurch in einem Winkel von mehr als 45°, vorzugsweise etwa 90° zueinander. Die Verbindungsstelle 16 ist besonders bevorzugt optisch in der Art eines Trichters ausgeführt, wie dies der Strahlverlauf in Fig. 3 verdeutlicht.

Aus der trichterförmigen Gestaltung der Verbindungsstelle 16 ergibt sich ein großer Vorteil auch für den optischen Rückweg, also den Weg des Fluoreszenzlichtes, welches von der Sensorfaser 01 zurück zum Detektor 21 geführt wird. Durch die gezeigte Strahlführung können die optischen Verluste minimiert werden bis zu dem Punkt, an dem der Anregungsarm in den Detektorarm mündet. Die vergleichsweise geringen Emissionen, die von den Fluoreszenzleuchtstoffen 02 ausgehen, lassen sich dadurch am Detektor besonders gut auswerten. Damit lässt sich zwischen dem Sensorfuß und der Sensorfaser ein Abstand von mehreren 100 μm realisieren, was für praxisrelevante Aufbauten von großem Vorteil ist.

Bevorzugt ist an der zur Sensorfaser 01 gewandten Seite des Sensorfußes 14 eine Linse 23 ausgebildet, vorzugsweise im Material des Sensorfußes integriert. Die Linse 23 liegt in Richtung der Anregungsstrahlung hinter der torischen Fläche, um die Anregungsstrahlung auf die Eingangsstirnfläche der Sensorfaser 01 zu fokussieren. Die Linse kann als eine sphärische oder asphärische Linse gebildet sein und ggf. entspiegelt sein.

Besonders bevorzugt ist die numerische Apertur, die sich nach der Linse 23 am Sensorfuß 14 in Richtung Sensorfaser 01 ergibt, derart angepasst, dass sie im Wesentlichen der numerische Apertur der Sensorfaser entspricht.

Gemäß einer bevorzugten Ausführung weitet sich der Anregungsarm 11 im Bereich der Verbindungsstelle 16 im Durchmesser auf, wie dies am Stahlverlauf in Fig. 3 verdeutlicht ist. Diese Aufweitung erfolgt vorzugsweise durch lineare, alternativ durch nicht-lineare Zunahme des Durchmessers in Richtung zum Sensorfuß 14. Die Mantellinien des sich im Längsschnitt ergebenden Kegels der Aufweitung können somit beliebig geformt sein.

Im Bereich der Krümmung des Sensorfußes erfolgt die Umlenkung der Strahlen, bevorzugt an einer torischen Fläche 24, die wie folgt mathematisch beschrieben werden kann:

$$z = \frac{C * y^2}{1 + \sqrt{1 - (C^2 * (1 + KK) * y^2)}}$$

wobei

- der erste Radius der torischen Fläche bezieht sich auf die X-Achse (Radius um eine Achse in X-Richtung) (R_um_X; C = 1/R_um_X);
- KK = konische Konstante;
- die Y-Koordinate wird in der Formel als y eingesetzt und es ergibt sich die Z-Koordinate für den Fall, dass die torische Fläche im Punkt Y=0 und Z=0 ihren Ursprung hat und nicht um 45° gegen den Uhrzeigersinn gedreht ist;
- der zweite Radius der torischen Fläche bezieht sich auf die Y-Achse (Radius um eine Achse in Y-Richtung), für den Fall, dass die torische Fläche nicht um 45° gegen den Uhrzeigersinn gedreht ist.

Die Reflexion erfolgt an der torischen Fläche vorzugsweise als Totalreflexion. Beispielsweise kann in diesem Bereich auch eine zusätzliche spiegelnde Schicht aufgetragen sein. Dies ist zweckmäßig, Falls die Brechzahl des Materials im Sensorfuß zu klein ist, dann ist keine Totalreflexion möglich. In diesem Fall kann eine zusätzliche spiegelnde Schicht an der torischen Fläche angebracht werden.

Durch diese Gestaltung wird sichergestellt, dass die Anregungsstrahlung vom Anregungsarm 11 über die Aufweitung im Bereich der Verbindungsstelle 16 und torische Fläche 24 bis zur Linse 23 in dem Material des Anregungsarms verläuft. Erst an der Linse 23 erfolgt ein Übergang zu Luft bzw. Gas und dann in die Sensorfaser 01.

**Bezugszeichen**

| | |
|---|---|
| 01 | Sensorfaser |
| 02 | Fluoreszenzleuchtstoffe |
| 03 | LED-Chip / LED |
| 04 | Koppelfaser |
| 05 | -- |
| 06 | Kleber |
| 07 | Trägerglas |
| 08 | Filterschichten |
| 09 | erstes Farbglasstück |
| 10 | -- |
| 11 | Anregungsarm |
| 12 | Y-Koppler |
| 13 | Detektorarm |
| 14 | Sensorfuß |
| 15 | -- |
| 16 | Verbindungsstelle |
| 17 | zweites Farbglasstück |
| 18 | Linse |
| 19 | Filter |
| 20 | -- |
| 21 | Detektor |
| 22 | Strahlteiler |
| 23 | asphärische Linse |
| 24 | torische Fläche |

**Patentansprüche**

1. Anordnung zum Betrieb eines Biosensors (01), der eine Strahlung emittiert, umfassend:

   - eine Anregungslichtquelle (03), welche mindestens eine Anregungsstrahlung für den Biosensor erzeugt;
   - eine Koppelfaser (04), an deren Eintrittsfläche die Anregungsstrahlung eingekoppelt wird;
   - einen optischen Detektor (21); und
   - einen optischen Y-Koppler (12) mit einem Anregungsarm (11), der an die Austrittsfläche der Koppelfaser (04) angeschlossen ist, einem Detektorarm (13), der an den optischen Detektor (21) angeschlossen ist, und einem Sensorfuß (14), welcher an den Biosensor (01) anschließbar ist;

wobei der Anregungsarm (11) eine konische Form aufweist, wobei die Strahlachse des Anregungsarms (11) mit der Hauptstrahlachse des Detektorarms (13) einen an einer Verbindungsstelle (16) zwischen dem Detektorarm (13) und dem Anregungsarm (11) eingeschlossenen Winkel im Bereich von 5° bis 70° einschließt, wobei der Durchmesser des Anregungsarms (11) an der Verbindungsstelle (16) mit dem Detektorarm (13) weniger als zwei Drittel des Durchmessers des Detektorarms (13) beträgt, und wobei der Anregungsarm (11) einen sich in Strahlungsrichtung verjüngenden Querschnitt aufweist.

2. Anordnung nach Anspruch 1, **dadurch gekennzeichnet, dass** der Durchmesser des Anregungsarms (11) an der Verbindungsstelle (16) weniger als halb so groß ist wie an seiner Eintrittsfläche.

3. Anordnung nach Anspruch 1 oder 2, **dadurch gekennzeichnet, dass** der Detektorarm (13) und der Sensorfuß (14) eine gemeinsame Hauptstrahlachse aufweisen.

4. Anordnung nach einem der Ansprüche 1 bis 3, **dadurch gekennzeichnet, dass** die Anregungslichtquelle ein LED-Chip (03) ist, dessen Emissionsebene von der Eintrittsfläche der Koppelfaser (04) in einem Abstand positioniert ist, der das 0,1 bis 10-fache des Durchmessers der Koppelfaser (04) beträgt.

5. Anordnung nach Anspruch 4, **dadurch gekennzeichnet, dass** die Koppelfaser (04) eine Länge besitzt, die das 7-fache bis 13-fache des Abstands zwischen der Anregungslichtquelle (03) und der Eintrittsfläche der Koppelfaser (04) beträgt, insbesondere im Bereich von 7 bis 13 mm liegt.

6. Anordnung nach Anspruch 4, **dadurch gekennzeichnet, dass** die Eintrittsfläche der Koppelfaser (04) ausgebildet ist als plane, sphärische, asphärische Fläche oder als Freiformfläche.

7. Anordnung nach einem der Ansprüche 1 bis 6, **dadurch gekennzeichnet, dass** zwischen der Austrittsfläche der Koppelfaser (04) und der Eintrittsfläche des Anregungsarms (11) ein Cut-off-Filter (07, 08) angeordnet ist, welcher die Wellenlänge der vom Biosensor (01) emittierbaren Strahlung aus der Anregungsstrahlung herausfiltert.

8. Anordnung nach einem der Ansprüche 1 bis 7, **dadurch gekennzeichnet, dass** die Koppelfaser (04) mit einer geradlinig verlaufenden Längsachse ausgebildet ist.

9. Anordnung nach Anspruch 7 oder nach dem auf Anspruch 7 rückbezogenen Anspruch 8, **dadurch gekennzeichnet, dass** der Cut-off-Filter aus einem Trägerglas (07) mit darauf aufgebrachten optischen Filterschichten (08) besteht, wobei die Austrittsfläche der Koppelfaser (04) vorzugsweise an den Filterschichten (08) angeklebt ist.

10. Anordnung nach einem der Ansprüche 1 bis 9, **dadurch gekennzeichnet, dass** zwischen der Austrittsfläche des Detektorarms (13) und dem Detektor (21) eine Linse (18) zur Kollimation der vom Biosensor (01) emittierten Strahlung angeordnet ist, wobei zwischen der Linse (18) und dem Detektor (21) ein optischer Filter (19) angeordnet ist, welcher eintreffende Anteile der Anregungsstrahlung blockiert.

11. Anordnung nach einem der Ansprüche 1 bis 10, **dadurch gekennzeichnet, dass** am austrittseitigen Ende des Detektorarms (13) ein Farbglasstück (17) in Wellenleiterform angeordnet ist.

12. Anordnung nach einem der Ansprüche 1 bis 11, **dadurch gekennzeichnet, dass** der Sensorfuß (14) einen gekrümmten Abschnitt umfasst, in welchem eine Strahlumlenkung mit einem Winkel von mehr als 45°, vorzugsweise etwa 90° erfolgt.

13. Anordnung nach Anspruch 12, **dadurch gekennzeichnet, dass** im Sensorfuß (14) eine strahlumlenkende, torische Fläche (24) ausgebildet ist.

14. Anordnung nach einem der Ansprüche 1 bis 13, **dadurch gekennzeichnet, dass** sie weiterhin zur Bestimmung des Glukosegehalts, insbesondere im Blut, ausgebildet ist und den Biosensor (01, 02) umfasst, der in ein Gewebe implantierbar ist und bei Anregung eine Strahlung emittiert.

15. Anordnung nach Anspruch 14, **dadurch gekennzeichnet, dass** der Biosensor als eine optische Faser (01) gebildet ist, welche an ihrer Austrittsfläche glukosesensitive Fluoreszenzleuchtstoffe (02) besitzt, die bei Anregung mit der Anregungsstrahlung eine Fluoreszenzstrahlung mit einer Fluoreszenzwellenlänge emittieren.

## Claims

1. An arrangement for operating a biosensor (01) emitting radiation, comprising:

- an excitation light source (03), which generates at least one excitation radiation for the biosensor;
- a coupling fiber (04), at the entry surface of which the excitation radiation is coupled in;
- an optical detector (21); and
- an optical Y-coupler (12) including an excitation arm (11), which is connected to the exit surface of the coupling fiber (04), a detector arm (13), which is connected to the optical detector (21), and a sensor foot (14), which can be connected to the biosensor (01);

wherein the excitation arm (11) having a conical shape, wherein the radiation axis of the excitation arm (11) forms an angle in the range of 5° to 70° with the main radiation axis of the detector arm (13) at a connecting point (16) between the detector arm (13) and the excitation arm (11), wherein the diameter of the excitation arm (11) at the connecting point (16) to the detector arm (13) being less than two thirds of the diameter of the detector arm (13), and wherein the excitation arm (11) has a cross-section tapering in the direction of radiation.

2. The arrangement according to claim 1, **characterized in that** the diameter of the excitation arm (11) at the connecting point (16) is less than half as large as at the entry surface thereof.

3. The arrangement according to claim 1 or 2, **characterized in that** the detector arm (13) and the sensor foot (14) have a shared main radiation axis.

4. The arrangement according to any one of claims 1 to 3, **characterized in that** the excitation light source is an LED chip (03), the emission plane of which is positioned at a distance of 0.1 to 10 times the diameter of the coupling fiber from the entry surface of the coupling fiber (04).

5. The arrangement according to claim 4, **characterized in that** the coupling fiber (04) has a length that is 7 times to 13 times the distance between the excitation light source (03) and the entry surface of the coupling fiber (04), in particular in the range of 7 to 13 mm.

6. The arrangement according to claim 4, **characterized in that** the entry surface of the coupling fiber (04) is designed as a planar, spherical, aspherical or free-form surface.

7. The arrangement according to any one of claims 1 to 6, **characterized in that** a cut-off filter (07, 08), which filters the wavelength of the radiation emittable by the biosensor (01) out of the excitation radiation, is arranged between the exit surface of the coupling fiber (04) and the entry surface of the excitation arm (11).

8. The arrangement according to any one of claims 1 to 7, **characterized in that** the coupling fiber (04) is designed with a rectilinearly extending longitudinal axis.

9. The arrangement according to claim 7 or according to claim 8 referenced to claim 7, **characterized in that** the cut-off filter is composed of a carrier glass (07) including optical filter layers (08) applied thereon, wherein the exit surface of the coupling fiber (04) preferably being glued to the filter layers (08).

10. The arrangement according to any one of claims 1 to 9, **characterized in that** a lens (18) is arranged between the exit surface of the detector arm (13) and the detector (21) for collimating the radiation emitted by the biosensor (01), wherein an optical filter (19), which blocks incoming fractions of the excitation radiation, is arranged between the lens (18) and the detector (21).

11. The arrangement according to any one of claims 1 to 10, **characterized in that** a colored glass piece (17) in waveguide form is arranged at the exit-side end of the detector arm (13).

12. The arrangement according to any one of claims 1 to 11, **characterized in that** the sensor foot (14) comprises a curved section, in which a beam deflection is carried out at an angle of more than 45°, and preferably of approximately 90°.

13. The arrangement according to claim 12, **characterized in that** a beam-deflecting, toric surface (24) is formed in the sensor foot (14).

14. An arrangement according to any one of claims 1 to 13, **characterized in that** it is still designed for determining the glucose content, in particular of blood, and comprising the biosensor (01, 02), which can be implanted into tissue and emits radiation upon excitation.;

15. The arrangement according to claim 14, **characterized in that** the biosensor is formed as an optical fiber (01), which includes glucose-sensitive fluorescent luminophores (02) at the exit surface thereof, which upon excitation by the excitation radiation emit fluorescent radiation having a fluorescent wavelength.

## Revendications

1. Agencement pour faire fonctionner un biocapteur (01), qui émet un rayonnement, comprenant :

- une source de lumière d'excitation (03), laquelle produit au moins un rayonnement d'excitation pour le biocapteur,
- une fibre d'accouplement (04), à la surface d'incidence de laquelle est injecté le rayonnement d'excitation,
- un détecteur optique (21), et
- un coupleur optique en Y (12) avec un bras d'excitation (11), qui est raccordé à la surface de sortie de la fibre d'accouplement (04), un bras de détecteur (13), qui est raccordé au détecteur optique (21) et un pied de capteur (14), lequel peut être raccordé au biocapteur (01),

sachant que le bras d'excitation (11) comporte une forme conique, sachant que l'axe de faisceau du bras d'excitation (11) forme avec l'axe de faisceau principal du bras de détecteur (13) un angle compris à un emplacement de liaison (16) entre le bras de détecteur (13) et le bras d'excitation (11) dans une plage de 5° à 70°, sachant que le diamètre du bras d'excitation (11) au point de raccordement (16) avec le bras de détecteur (13) comporte moins de deux tiers du diamètre du bras de détecteur (13) et sachant que le bras d'excitation (11) comporte une section transversale se réduisant en direction du rayonnement.

2. Agencement selon la revendication 1, **caractérisé en ce que** le diamètre du bras d'excitation (11) au point de raccordement (16) est inférieur de la moitié de sa surface d'incidence.

3. Agencement selon la revendication 1 ou 2, **caractérisé en ce que** le bras de détecteur (13) et le pied de capteur (14) comportent un axe de faisceau principal commun.

4. Agencement selon l'une quelconque des revendications 1 à 3, **caractérisé en ce que** la source de lumière d'excitation (03) est une puce à DEL (diodes électroluminescentes), dont le plan d'émission de la surface d'incidence de la fibre d'accouplement (04) est positionné à une distance qui est de 0,1 à 10 fois le diamètre de la fibre d'accouplement (04).

5. Agencement selon la revendication 4, **caractérisé en ce que** la fibre d'accouplement (04) possède une longueur, qui comporte 7 fois à 13 fois la distance entre la source de lumière d'excitation (03) et la surface d'incidence de la fibre d'accouplement (04), se situe en particulier dans une plage de 7 à 13 mm.

6. Agencement selon la revendication 4, **caractérisé en ce que** la surface d'incidence de la fibre d'accouplement (04) est constituée sous la forme d'une surface plane, sphérique, asphérique ou sous la forme d'une surface de forme libre.

7. Agencement selon l'une quelconque des revendications 1 à 6, **caractérisé en ce qu'**un filtre de coupure (07, 08) est disposé entre la surface de sortie de la fibre d'accouplement (04) et la surface d'incidence du bras d'excitation (11), lequel exfiltre du rayonnement d'excitation la longueur d'ondes du rayonnement pouvant être émis par le biocapteur (01).

8. Agencement selon l'une quelconque des revendications 1 à 7, **caractérisé en ce que** la fibre d'accouplement (04) est constituée avec un axe longitudinal passant en ligne droite.

9. Agencement selon la revendication 7 ou selon la revendication 8 dépendante de la revendication 7, **caractérisé en ce que** le filtre de coupure est composé d'un verre porteur (07) avec des couches de filtrage (08) optiques appliquées dessus, sachant que la surface de sortie de la fibre d'accouplemené (04) est collée de préférence sur les couches de filtrage (08).

10. Agencement selon l'une quelconque des revendications 1 à 9, **caractérisé en ce qu'**une lentille (18) de collimation du rayonnement émis par le biocapteur (01) est disposée entre la surface de sortie du bras de détecteur (13) et le détecteur (21), sachant qu'un filtre optique (19) est disposé entre la lentille (18) et le détecteur (21), lequel bloque les parties incidentes du rayonnement d'excitation.

11. Agencement selon l'une quelconque des revendications 1 à 10, **caractérisé en ce qu'**un élément de verre coloré (17) dans la forme de guide d'ondes est disposé à l'extrémité du bras de détecteur (13) du côté sortie.

12. Agencement selon l'une quelconque des revendications 1 à 11, **caractérisé en ce que** le pied de capteur (14) comprend une section courbe dans laquelle a lieu une déviation de rayonnement avec un angle de plus de 45°, de préférence à peu près 90°.

13. Agencement selon la revendication 12, **caractérisé en ce qu'**une surface torique (24) déviant le rayonnement est constituée dans le pied de capteur (14).

14. Agencement selon l'une quelconque des revendications 1 à 13, **caractérisé en ce qu'**il est constitué en plus pour déterminer la teneur en glucose, en particulier dans le sang et comprend le biocapteur (01, 02), qui peut être implanté dans un tissu et émet un rayonnement lors de l'excitation.

**EP 4 070 077 B1**

**15.** Agencement selon la revendication 14, **caractérisé en ce que** le biocapteur est formé sous la forme d'une fibre optique (01), laquelle possède sur sa surface de sortie des substances électroluminescentes sensibles au glucose (02), qui émettent lors de l'excitation avec le rayonnement d'excitation un rayonnement fluorescent avec une longueur d'onde de fluorescence.

Fig. 1

Fig. 2

EP 4 070 077 B1

12

Fig. 3

EP 4 070 077 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102015101847 B4
- DE 69408976 T2
- US 4344438 A
- WO 2014116597 A1
- EP 2989975 A1
- DE 4322734 A1
- DE 4341086 A1
- US 6553164 B
- DE 69414139 T2
- US 20040072358 A1

**In der Beschreibung aufgeführte Nicht-Patentliteratur**

- **MÜLLER AJ** ; **KNUTH M** ; **NIKOLAUS KS** ; **KRIVÄNEK R** ; **KÜSTER F** ; **HASSLACHER C**. First clinical evaluation of a new percutaneous optical fiber glucose sensor for continuous glucose monitoring in diabetes. *Journal of Diabetes Science and Technology 2013*, 01 January 2013, vol. 7 (1), 13-23